# EUROPEAN PATENT SPECIFICATION

(11) **EP 2 134 341 B1**
(45) Date of publication and mention of the grant of the patent: **17.06.2015**
(21) Application number: 08725389.4
(22) Date of filing: 08.02.2008
(51) Int. Cl.: A61K 31/454, A61K 31/435, A61P 35/00

(54) **METHOD OF TREATING CELL PROLIFERATIVE DISORDERS USING GROWTH HORMONE SECRETAGOGUES**
VERFAHREN ZUR BEHANDLUNG VON ZELLPROLIFERATIONSSTÖRUNGEN MIT WACHSTUMSHORMON-SEKRETAGOGEN
PROCÉDÉ DE TRAITEMENT DES TROUBLES PROLIFÉRATIFS CELLULAIRES UTILISANT UN SÉCRÉTAGOGUE D'HORMONE DE CROISSANCE

(30) Priority: 13.02.2007 US 901609 P
(43) Date of publication of application: 23.12.2009
(73) Proprietor: Helsinn Healthcare S.A., 6912 Lugano / Pazzallo (CH)
(72) Inventor: POLVINO, Williams, J., Tinton Falls, NJ 07724 (US)
(74) Representative: Zardi, Marco
(86) International application number: PCT/US2008/001748
(87) International publication number: WO 2008/100448

(56) References cited:
- WO-A1-02/070511
- WO-A1-2005/097261
- WO-A1-2008/130464
- US-A- 6 124 263
- US-A1- 2007 021 331
- US-B1- 6 303 620
- US-B1- 6 566 337
- US-B1- 6 919 315
- US-B2- 6 576 648
- GARCIA JOSE M ET AL: "Effect on body weight and safety of RC-1291, a novel, orally available ghrelin mimetic and growth hormone secretagogue: results of a phase I, randomized, placebo-controlled, multiple-dose study in healthy volunteers.", THE ONCOLOGIST MAY 2007 LNKD- PUBMED:17522248, vol. 12, no. 5, May 2007 (2007-05), pages 594-600, XP002648029, ISSN: 1083-7159

## Description

### RELATED APPLICATIONS

This application claims the benefit of United States provisional patent application number 60/901,609, filed February 13, 2007.

### BACKGROUND OF THE INVENTION

Cancer is a leading cause of death in developed countries. Despite continuing advances in both diagnosis and treatment regimens, most existing treatments have undesirable side effects and limited efficacy. Progress in this field has been hindered because a number of different cellular events contribute to the formation of tumors, and many of these events are still not well understood. Chemotherapy is one of the major options available for the treatment of cancers. However, effective chemotherapeutic agents are still needed to treat the increasing numbers of subjects developing cancer.

Insulin-like growth factor binding protein-3 (IGFBP-3) is the major circulating carrier protein for IGFs and is also active in the cellular environment as a potent antiproliferative agent (Baxter, R.C. (2001) Mol. Pathol. 54(3): 145-148).

Accordingly, therapeutics that are able to differentially increase the levels of IGFBP-3 would be valuable anticancer agents.

WO-A-2005/097261 discloses a family of compounds, including RC-1291, active in reducing C-reactive protein levels in a subject, and their use in the treatment of inflammation in a variety of conditions. US-A-2007/0021331 enumerates various compounds, including RC-1291, as growth hormone secretagogues. US-A-6124263 shows compounds e.g. hexarelin, Ala-hexarelin, having affinity for tumour membranes and being proposed for inhibiting the growth of tumorigenic cells.

### SUMMARY OF THE INVENTION

The present invention relates to treatments of subjects having cancer. The treatments comprise administering to a subject in need thereof a therapeutically effective amount of a growth hormone secretagogue compound or a pharmaceutically acceptable salt, hydrate or solvate thereof.

The invention provides a treatment for cancer in a subject in need thereof, by administering to the subject an effective amount of a growth hormone secretagogue.

The growth hormone secretagogue is represented by the structural Formula III 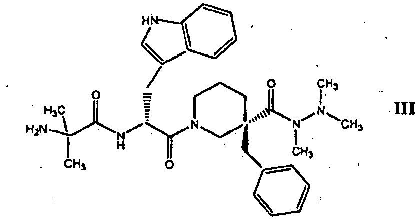 or a pharmaceutically acceptable salt, solvate or hydrate thereof.

The compound of Formula III is fully described in U.S. Patent No. 6,576,648 to Hansen, et al. The chemical name of the compound of Formula III is 2-Amino-N-{(1R)-2-[3-benzyl- 3-(N,N',N'-trimethylhydrazinocarbonyl)piperidin-1-yl]-1((1H-indol-3-yl)-2-oxoethyl}-2-methylpropionamide, and is referred to as RC-1291 and anamorelin.

The invention further provides pharmaceutical compositions comprising the growth hormone secretagogue of the invention for the treatment of cancer. The pharmaceutical compositions may further comprise one or more additional anti-cancer agents.

### BRIEF DESCRIPTION OF THE DRAWINGS

FIG. 1 depicts the results of a twelve week study indicating administration of the growth hormone secretagogue of Formula III results in significantly increased levels of IGFBP-3 and IGF-1.
FIG. 2 depicts the results of a study demonstrating the increase in levels of IGF-1 in subjects administered RC-1291 as compared to a placebo.
FIG. 3 depicts the results of a study demonstrating the increase in levels of IGFBP-3 in subjects administered RC-1291 as compared to a placebo.
Figure 4 sets forth Table 4, a summary of IGF-1 baseline levels and levels at day 7.
Figure 5 sets forth Table 5, a summary of IGF-1 levels for day 14 and week 4.
Figure 6 sets forth Table 6, a summary of IGF-1 levels at weeks 4, 8 and 12.
Figure 7 sets forth Table 7, a summary of IGFBP-3 baseline levels and levels at day 7.
Figure 8 sets forth Table 8, a summary of IGFBP-3 levels for day 14 and week 4.
Figure 9 sets forth Table 9, a summary of IGFBP-3 levels at weeks 4, 8 and 12.
Figure 10 depicts the change in total body mass for subjects administered RC-1291 and a placebo.
Figure 11 depicts the change in lean body mass for subjects administered RC-1291 and a placebo.
Figure 12 depicts the change in body weight for subjects administered 50 and 100mg dosages of RC-1291 and a placebo.

The foregoing and other objects, features and advantages of the invention will be apparent from the following more particular description of preferred embodiments of the invention.

### DETAILED DESCRIPTION OF THE INVENTION

The present invention relates to treatments of subjects having cell proliferative disorders, i.e. cancer. The treatments comprise administering to a subject in need thereof a therapeutically effective amount of a growth hormone secretagogue compound of formula III or a pharmaceutically acceptable salt, hydrate or solvate thereof.

The invention also provides treatments of cancer by administering a therapeutically effective amount of said growth hormone secretagogue compound or a pharmaceutically acceptable salt, hydrate or solvate thereof, further comprise administering one or more additional anticancer agents. The additional anticancer agent or agents may be selected from, acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-n1; interferon alfa-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine, mechlorethamine oxide hydrochloride rethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride, improsulfan, benzodepa, carboquone, triethylenemelamine, triethylenephosphoramide, triethylenethiophosphoramide, trimethylolomelamine, chlornaphazine, novembichin, phenesterine, trofosfamide, estermustine, chlorozotocin, gemzar, nimustine, ranimustine, dacarbazine, mannomustine, mitobronitol,aclacinomycins, actinomycin F(1), azaserine, bleomycin, carubicin, carzinophilin, chromomycin, daunorubicin, daunomycin, 6-diazo-5-oxo-1-norleucine, doxorubicin, olivomycin, plicamycin, porfiromycin, puromycin, tubercidin, zorubicin, denopterin, pteropterin, 6-mercaptopurine, ancitabine, 6-azauridine, carmofur, cytarabine, dideoxyuridine, enocitabine, pulmozyme, aceglatone, aldophosphamide glycoside, bestrabudil, defofamide, demecolcine, elfornithine, elliptinium acetate, etoglucid, flutamide, hydroxyurea, lentinan, phenamet, podophyllinic acid, 2-ethylhydrazide, razoxane, spirogermanium, tamoxifen, taxotere, tenuazonic acid, triaziquone, 2,2',2"-trichlorotriethylamine, urethan, vinblastine, vincristine, vindesine and related agents. 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP- DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cisporphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1-receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid

A+myobacterium cell wall sk; mopidamol; multiple drug resistance gene inhibitor; multiple tumor suppressor 1 -based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; oracin; oral cytokine inducer; ormaplatin; osaterone; oxaliplatin; oxaunomycin; taxel; taxel analogues; taxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stemcell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer. Additional cancer therapeutics include monoclonal antibodies such as rituximab, trastuzumab and cetuximab.

### GROWTH HORMONE SECRETAGOGUES/GHRELIN AGONISTS

As used herein, "growth hormone secretagogue" refers to a substance (e.g., a molecule, a compound) which promotes (induces or enhances) at least one function characteristic of a growth hormone secretagogue receptor (GHS receptor).

Exemplary growth hormones secretagogues are ghrelins mimetics such as ghrelin agonists. In one example, the growth hormone secretagogue compound or ghrelin agonist binds the GHS receptor or ghrelin receptor (i.e., is a ghrelin or GHS receptor agonist) and induces the secretion of growth hormone. A compound having GHS receptor agonist activity (e.g., a GHS receptor or ghrelin receptor agonist) can be identified and activity assessed by any suitable method. For example, the binding affinity of a GHS receptor agonist to the GHS receptor can be determined employing receptor binding assays and growth hormone stimulation can be assessed as described in U.S. Patent No. 6,919,315.

GHS receptors and ghrelin receptors are expressed in the hypothalamus, pituitary and pancreas, among other tissues. Activation of these receptors in the pituitary induces the secretion of growth hormone. In addition to inducing the secretion of growth hormone, recent studies have shown the growth hormone secretagogues can increase appetite and body weight. At typical doses, growth hormone secretagogues are also known to induce the secretion of IGF-1.

The growth hormone secretagogue used in the present invention is: 2-Amino-N-{(1R)-2-[3-benzyl-3-(N,N',N'-trimethylhydrazinocarbonyl)piperidin-1-yl]-1-((1H-indol-3-yl)-2-oxoethyl}-2-methylpropionamide, represented by structural Formula III: 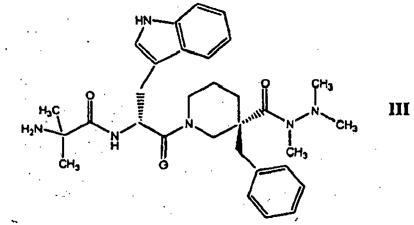 and pharmaceutically acceptable salts, hydrates or solvates thereof.

As used herein, the term "cell proliferative disorder" is used to mean a condition in which a cell in a subject's body undergoes abnormal, uncontrolled proliferation. Thus, "cancer" is a type of cell-proliferative disorder. Examples of cancers include, solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, nile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenroglioma, schwannoma, meningioma, melanoma, neuroblastoma, and retinoblastoma). Lymphoproliferative disorders are also considered to be proliferative diseases. Preferred cancers include lung, colon, and rectal cancers. The terms "cancer," "neoplasm," and "tumor," are used interchangeably and in either the singular or plural form, refer to cells that have undergone a malignant transformation that makes them pathological to the host organism.

"Subject", as used herein, refers to animals such as mammals, including, primates (e.g., humans), cows, sheep, goats, horses, pigs, dogs, cats, rabbits, guinea pigs, rats, mice or other bovine, ovine, equine, canine, feline, rodent or murine species. In a preferred embodiment, the mammal is a human.

As used herein, "treating" and "treatment" refer to reducing or eliminating cancerous cells from a subject.

As used herein, "therapeutically effective amount" refers to an amount sufficient to elicit the desired biological response. In the present invention, the desired biological response is treating cell proliferative disorders, i.e., cancer.

The therapeutically effective amount or dose will depend on the age, sex and weight of the patient, and the current medical condition of the patient. The skilled artisan will be able to determine appropriate dosages depending on these and other factors to achieve the desired biological response.

A suitable dose per day for the growth hormone secretagogue can be in the range of from 1 ng to 10,000 mg, about 5 ng to about 9,500 mg, about 10 ng to about 9,000 mg, about 20 ng to about 8,500 mg, about 30 ng to about 7,500 mg, about 40 ng to about 7,000 mg, about 50 ng to about 6,500 mg, about 100 ng to about 6,000 mg, about 200 ng to about 5,500 mg, about 300 ng to about 5,000 mg, about 400 ng to about 4,500 mg, about 500 ng to about 4,000 mg, about 1 µg to about 3,500 mg, about 5 µg to about 3,000 mg, about 10 µg to about 2,600 mg, about 20 µg to about 2,575 mg, about 30 µg to about 2,550 mg, about 40 µg to about 2,500 mg, about 50 µg to about 2,475 mg, about 100 µg to about 2,450 mg, about 200 µg to about 2,425 mg, about 300 µg to about 2,000, about 400 µg to about 1,175 mg, about 500 µg to about 1,150 mg, about .5 mg to about 1,125 mg, about 1 mg to about 1,100 mg, about 1.25 mg to about 1,075 mg, about 1.5 mg to about 1,050 mg, about 2.0 mg to about 1,025 mg, about 2.5 mg to about 1,000 mg, about 3.0 mg to about 975 mg, about 3.5 mg to about 950 mg, about 4.0 mg to about 925 mg, about 4.5 mg to about 900 mg, about 5 mg to about 875 mg, about 10 mg to about 850 mg, about 20 mg to about 825 mg, about 30 mg to about 800 mg, about 40 mg to about 775 mg, about 50 mg to about 750 mg, about 100 mg to about 725 mg, about 200 mg to about 700 mg, about 300 mg to about 675 mg, about 400 mg to about 650 mg, about 500 mg, or about 525 mg to about 625 mg.

Other suitable doses per day for the growth hormone secretagogue include doses of about or greater than 1 ng, about 5 ng, about 10 ng, about 20 ng, about 30 ng, about 40 ng, about 50 ng, about 100 ng, about 200 ng, about 300 ng, about 400 ng, about 500 ng, about 1 µg, about 5 µg, about 10 µg, about 20 µg, about 30 µg, about 40 µg, about 50 µg, about 100 µg, about 200 µg, about 300 µg, about 400 µg, about 500 µg (0.5 mg), about 1 mg, about 1.25 mg, about 15 mg, about 2.0 mg, about 2.5 mg, about 3.0 mg, about 3.5 mg, about 4.0 mg, about 4.5 mg, about 5 mg, about 10 mg, about 20 mg, about 30 mg, about 40 mg, about 50 mg, about 100 mg, about 200 mg, about 300 mg, about 400 mg, about 500 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1125 mg, about 1150 mg, about 1175 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, about 1500 mg, about 1525 mg, about 1550 mg, about 1575 mg, about 1600 mg, about 1625 mg, about 1650 mg, about 1675 mg, about 1700 mg, about 1725 mg, about 1750 mg, about 1775 mg, about 1800 mg, about 1825 mg, about 1850 mg, about 1875 mg, about 1900 mg, about 1925 mg, about 1950 mg, about 1975 mg, about 2000 mg, about 2025 mg, about 2050 mg, about 2075 mg, about 2100 mg, about 2125 mg, about 2150 mg, about 2175 mg, about 2200 mg, about 2225 mg, about 2250 mg, about 2275 mg, about 2300 mg, about 2325 mg, about 2350 mg, about 2375 mg, about 2400 mg, about 2425 mg, about 2450 mg, about 2475 mg, about 2500 mg, about 2525 mg, about 2550 mg, about 2575 mg, about 2600 mg, about 3,000 mg, about 3,500 mg, about 4,000 mg, about 4,500 mg, about 5,000 mg, about 5,500 mg, about 6,000 mg, about 6,500 mg, about 7,000 mg, about 7,500 mg, about 8,000 mg, about 8,500 mg, about 9,000 mg, or about 9,500 mg.

A suitable dose of the growth hormone secretagogue can be In the range of from 1-150 mg per day, such as from about 10 mg to about 100 mg, for example, from about 20 mg to about 80 mg, such as about 30 mg to about 60 mg per day, such as about 50 mg per day. Preferred dosages are between 50 and 100 mg/day. The dose can be administered in a single dosage or in multiple dosages, for example from 1 to 4 or more times per day. When multiple dosages are used, the amount of each dosage can be the same or different.

A suitable dose for the additional therapeutic agent can be In same range as described above for the growth hormone secretagogue. The dose of growth hormone secretagogue and additional agent can be the same or different. Suitable doses for the additional agents can be found in the literature.

### Cancer Cachexia

Subjects that have cancer also commonly have cachexia. Cachexia Is a progressive loss of body weight, which is mainly due to loss of fat and skeletal muscle. Survival of cancer patients is directly related to the total weight loss and also the rate of weight loss. The instant invention provides methods of Inhibiting and reversing cachexia in subjects that have cancer. Specifically, the Instant Invention provides methods for treating a patient with cancer cachexia by administering to the subject a growth hormone secretagogue. As demonstrated in Example 3, both the lean body mass and total body weight increase in subjects administered RC-1291 as compared to a control group.

The treatment of cancer cachescia does however not forme part of the present invention.

### COMBINATION ADMINISTRATION

Administration of a growth hormone secretagogue can take place prior to, after or at the same time as treatment with an additional therapeutic agent, such as, for example, one or more additional anticancer agents, such as those disclosed herein.

### PHARMACEUTICAL COMPOSITIONS AND MODES OF ADMINISTRATION

The growth hormone secretagogue (also referred to herein as "active compound") of the invention can be incorporated into pharmaceutical compositions. Such compositions typically include the growth hormone secretagogue and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

The pharmaceutical compositions can be included in a container, pack, or dispenser together with instructions for administration.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. The compounds for use in the method of the invention can be formulated for administration by any suitable route, such as for oral or parenteral, for example, transdermal, transmucosal (e.g., sublingual, lingual, (trans)buccal), vaginal (e.g., trans- and perivaginally), (intra)nasal and (trans)rectal), subcutaneous, intramuscular, intradermal, intraarterial, intravenous, inhalation, and topical administration.

Suitable compositions and dosage forms include tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays, dry powders or aerosolized formulations.

It is preferred that the compounds are orally administered. Suitable oral dosage forms include, for example, tablets, capsules or caplets prepared by conventional means with pharmaceutically acceptable excipients such as binding agents (e.g., polyvinylpyrrolidone or hydroxypropylmethylcellulose); fillers (e.g., lactose, microcrystalline cellulose or calcium phosphate); lubricants (e.g., magnesium stearate, talc or silica); disintegrants (e.g., sodium starch glycollate); or wetting agents (e.g., sodium lauryl sulphate). If desired, the tablets can be coated, e.g., to provide for ease of swallowing or to provide a delayed release of active, using suitable methods. Liquid preparation for oral administration can be in the form of solutions, syrups or suspensions. Liquid preparations (e.g., solutions, suspensions and syrups) are also suitable for oral administration and can be prepared by conventional means with pharmaceutically acceptable additives such as suspending agents (e.g., sorbitol syrup, methyl cellulose or hydrogenated edible fats); emulsifying agent (e.g., lecithin or acacia); non-aqueous vehicles (e.g., almond oil, oily esters or ethyl alcohol); and preservatives (e.g., methyl or propyl p-hydroxy benzoates or sorbic acid).

As used herein, the term "pharmaceutically acceptable salt" refers to a salt of a compound to be administered prepared from pharmaceutically acceptable non-toxic acids including inorganic acids, organic acids, solvates, hydrates, or clathrates thereof. Examples of such inorganic acids are hydrochloric, hydrobromic, hydroiodic, nitric, sulfuric, and phosphoric. Appropriate organic acids may be selected, for example, from aliphatic, aromatic, carboxylic and sulfonic classes of organic acids, examples of which are formic, acetic, propionic, succinic, camphorsulfonic, citric, fumaric, gluconic, isethionic, lactic, malic, mucic, tartaric, para-toluenesulfonic, glycolic, glucuronic, maleic, furoic, glutamic, benzoic, anthranilic, salicylic, phenylacetic, mandelic, embonic (pamoic), methanesulfonic, ethanesulfonic, pantothenic, benzenesulfonic (besylate), stearic, sulfanilic, alginic, galacturonic, and the like.

The growth hormone secretagogues disclosed can be prepared in the form of their hydrates, such as hemihydrate, monohydrate, dihydrate, trihydrate, tetrahydrate and the like and as solvates.

It is understood that growth hormone secretagogue compounds can be identified, for example, by screening libraries or collections of molecules using suitable methods. Another source for the compounds of interest are combinatorial libraries which can comprise many structurally distinct molecular species.

Combinatorial libraries can be used to identify lead compounds or to optimize a previously identified lead. Such libraries can be manufactured by well-known methods of combinatorial chemistry,and screened by suitable methods.

### STEREOCHEMISTRY

If desired, a chiral carbon can be designated with an asterisk (*). When bonds to the chiral carbon are depicted as straight lines in the formulas of the invention, it is understood that both the (R) and (S) configurations of the chiral carbon, and hence both enantiomers and mixtures thereof, are embraced within the formula. As is used in the art, when it is desired to specify the absolute configuration about a chiral carbon, one of the bonds to the chiral carbon can be depicted as a wedge (bonds to atoms above the plane) and the other can be depicted as a series or wedge of short parallel lines is (bonds to atoms below the plane). The Cahn-Inglod-Prelog system can be used to assign the (R) or (S) configuration to a chiral carbon.

The compound of the present invention has two chiral carbons, it can have more than two optical isomers and can exist in diastereoisomeric forms. The compound can have up to 4 optical isomers and 2 pairs of enantiomers ((S,S)/(R,R) and (R,S)/(S,R)). The pairs of enantiomers (e.g., (S,S)/(R,R)) are mirror image stereoisomers of one another. The stereoisomers which are not mirror-images (e.g., (S, S) and (R,S)) are diastereomers. The diastereoisomeric pairs may be separated by methods known to those skilled in the art, for example chromatography or crystallization and the individual enantiomers within each pair may be separated as described above. The present invention includes each diastereoisomer of such compound and mixtures thereof.

### EXEMPLIFICATION

The present invention will now be illustrated by the following Examples. .

### Example 1

A double blind, placebo controlled, randomized study was designed to investigate the ability of growth hormone secretagogues to be used for the treatment of cell proliferative disorders.

Subjects were separated in to two groups: the placebo group and the RC-1291 group. The RC-1291 group received 50 mg of RC-1291 in two 25 mg capsules daily. The capsules were microcrystalline cellulose, magnesium stearate and RC-1291 HCl (25 mg) in a hard gelatin capsule. The placebo group received two placebo capsules daily. The placebo capsule was microcrystalline cellulose in a hard gelatin capsule.

One hour before breakfast subjects were administered the two capsules after fasting during the night.

The participants in the study are set forth in Table 1.

| | Placebo (PBO) N=28 | RC-1291 N=32 |
|---|---|---|
| Gender M/F | 16/12 | 21/11 |
| Age | 64.7 | 64.8 |
| Caucasian | 22 (78.6%) | 24 (75%) |
| Lung Cancer | 7 | 6 |
| Colorectal Cancer | 6 | 9 |
| IGF-1 change from baseline (nM) at 4 weeks | -0.56609 | 6.625703 |
| IGF-1 change from baseline (nM) at 8 weeks | -0.1595 | 4.728723 |
| IGF-1 change from baseline (nM) at 12 weeks | 0.130736 | 5.046411 |
| IGFBP-3 change from baseline (nM) at 4 weeks | -2.62295 | 26.88525 |
| IGFBP-3 change from baseline (nM) at 8 weeks | -0.32787 | 17.70492 |
| IGFBP-3 change from baseline (nM) at 12 weeks | -1.31148 | 20.65574 |

At various points over the course of the three month study, levels of IGFBP-3 and IGF-1 were measured in both the placebo and RC-1291 groups.

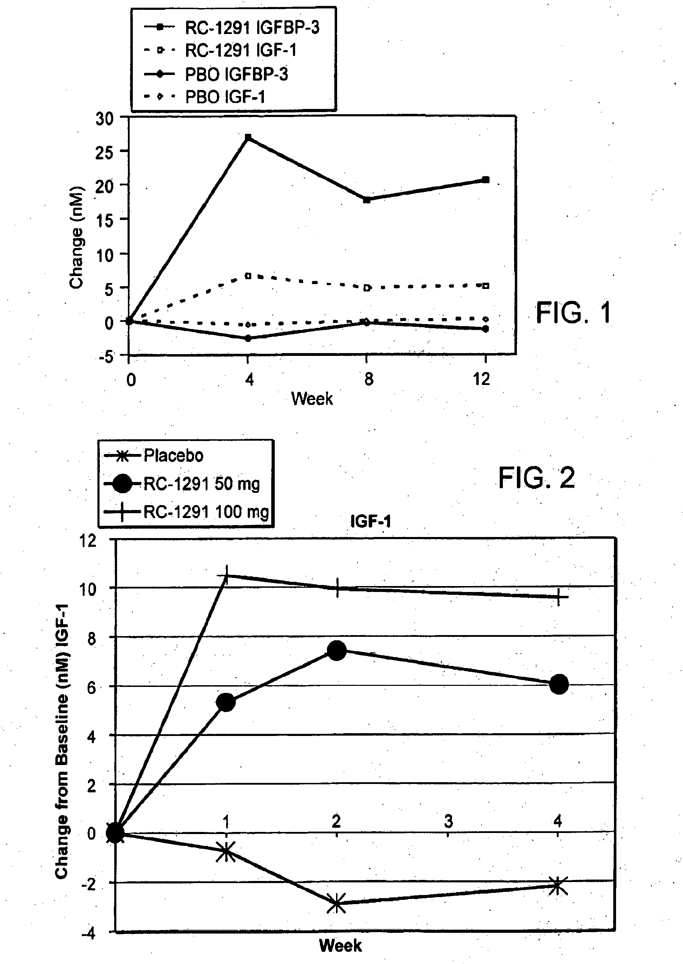

The results are presented in Figure 1 and Table 1. RC-1291 raises the levels of both IGFBP-3 and IGF-1 in a statistically significant manner. The effects of RC-1291 on IFGBP-3 were greater than 3 fold, on a molar basis, than on IGF-1. The increase in the molar ratio of IFGBP-3/IGF-1 suggests that RC-1291 exhibits antitumor potential.

Insulin-like growth factor binding protein-3 (IGFBP-3) is the major circulating carrier protein for IGFs and is also active in the cellular environment as a potent antiproliferative agent (Baxter, R.C. (2001) Mol.Pathol. 54(3):145-148). Accordingly, the results presented in this Example demonstrate that growth hormone secretagogues such as RC-1291 effectively raise the level of IGFBP-3 when administered to a subject and therefore will be useful for the treatment of cell proliferative disorders, e.g., cancer.

### Example 2

A second double blind, placebo controlled, randomized study was designed to investigate the ability of growth hormone secretagogues to be used for the treatment of cell proliferative disorders.

The participants in the study are set forth in Table 2.

| | Placebo (PBO) N=16 | RC-1291 50 mg N=16 | RC-1291 100 mg N=20 |
|---|---|---|---|
| Gender M/F | 6/10 | 10/6 | 15/5 |
| Age | 66.0 | 68.6 | 64.7 |
| Caucasian | 11(68.8%) | 15 (93.8%) | 14(70.0%) |
| Lung Cancer | 4 | 3 | 5 |
| Colorectal Cancer | 3 | 2 | 4 |

Subjects were separated into groups and administered a placebo or RC-1291 (Formula III). Each group was administered the appropriate number of capsules. The capsules were microcrystalline cellulose, magnesium stearate and RC-1291 HCl (25 mg) in a hard gelatin capsule. The placebo capsule was microcrystalline cellulose in a hard gelatin capsule. The RC-1291 groups received 50 mg or 100 mg of RC-1291 daily.

Serum was obtained at office visits, clearly labeled, and stored frozen at -20°C prior to shipment on dry ice to CRL, Lenexa, KS for analysis. Samples were assayed for IGF-1 and IGFBP-3.

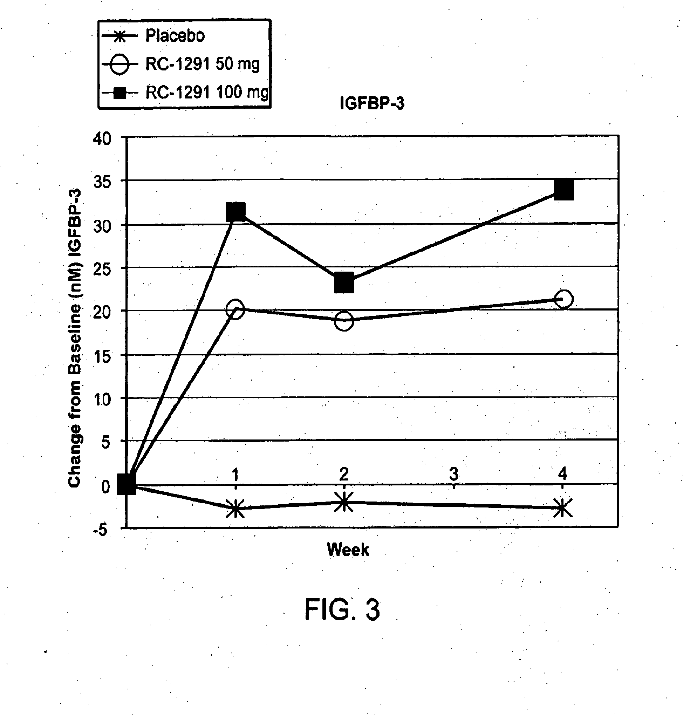

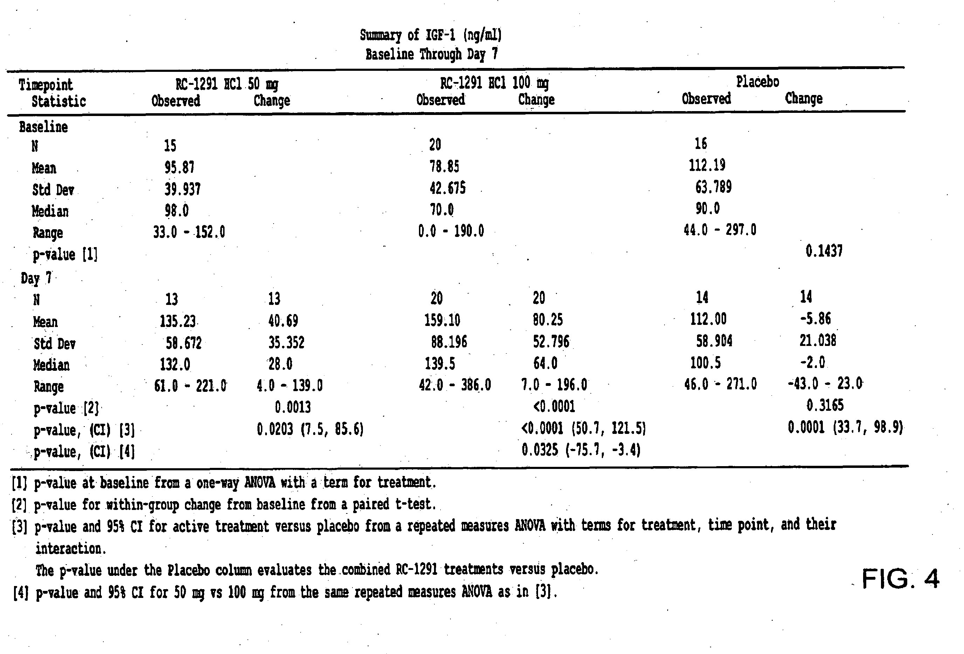

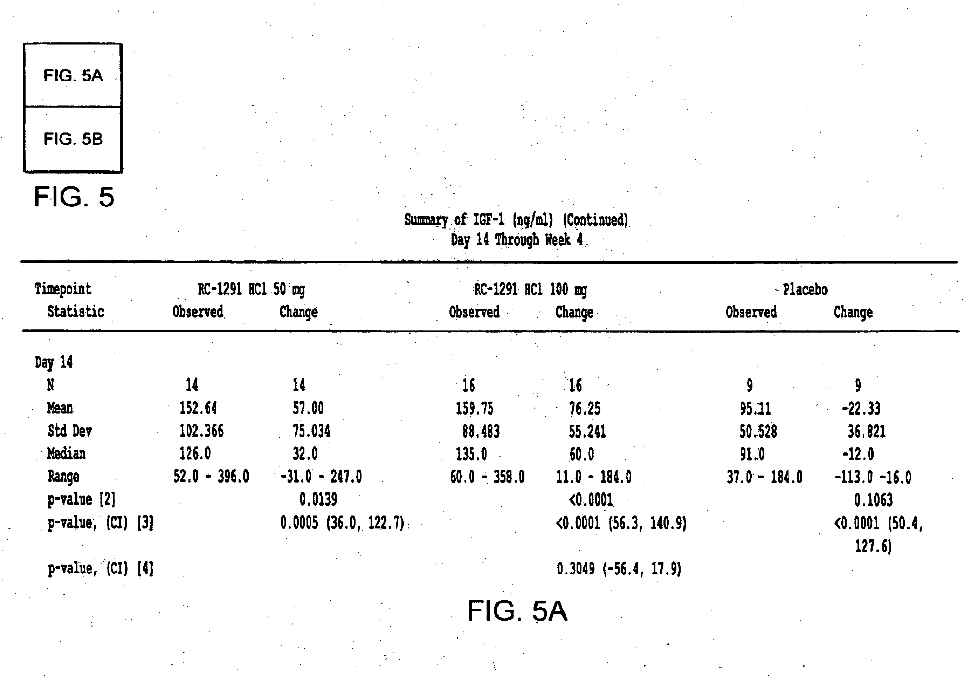

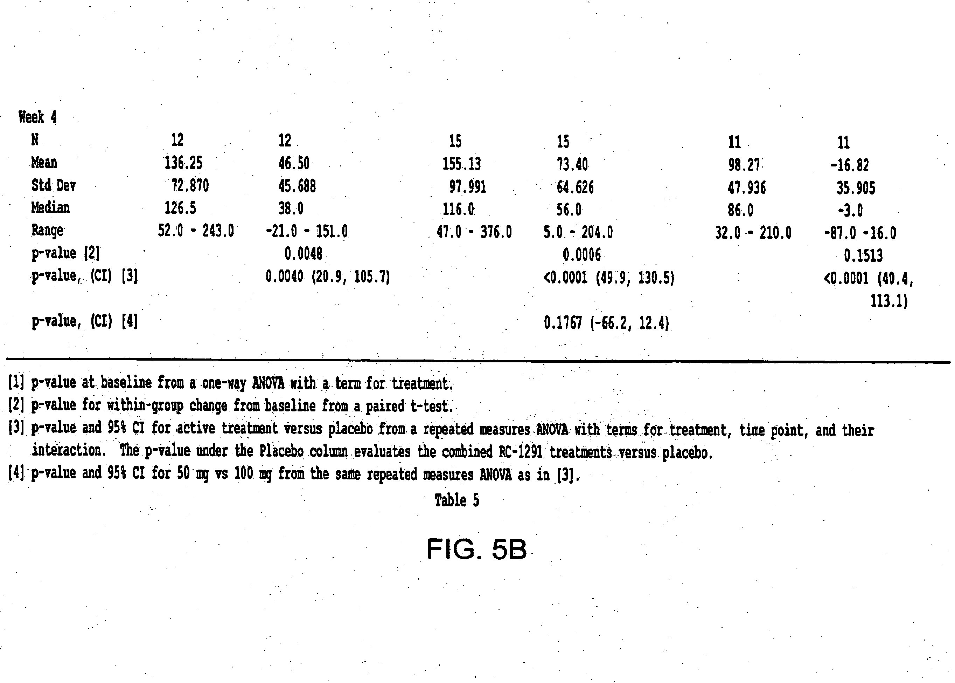

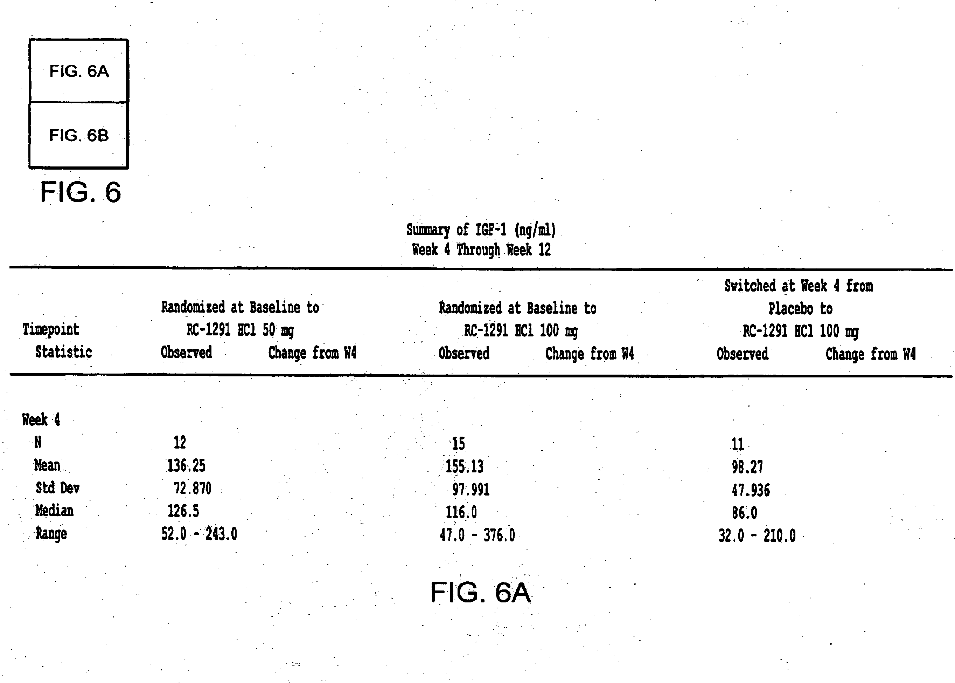

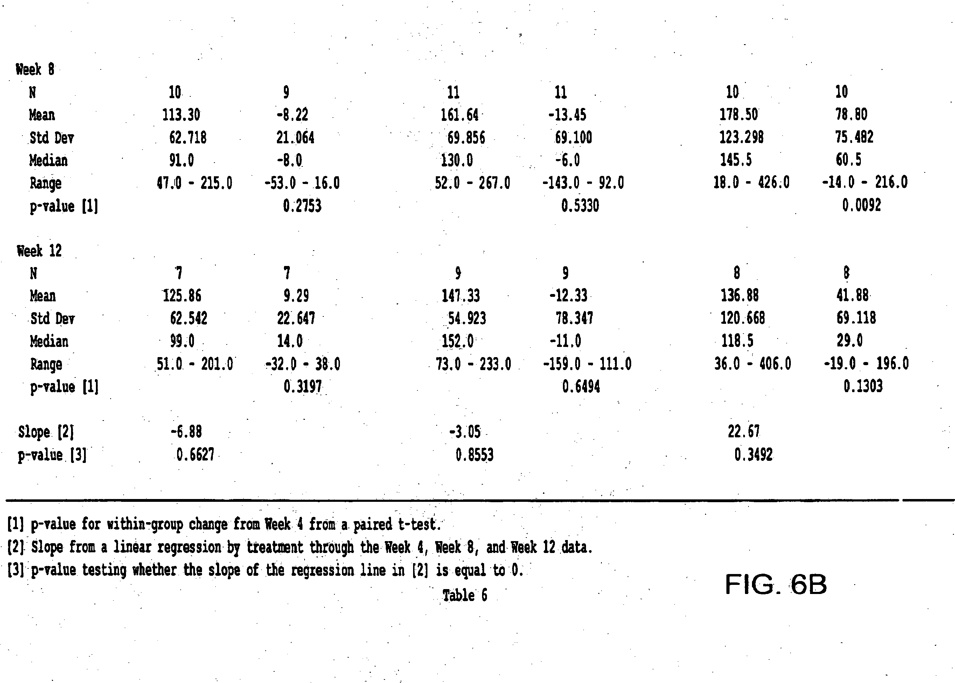

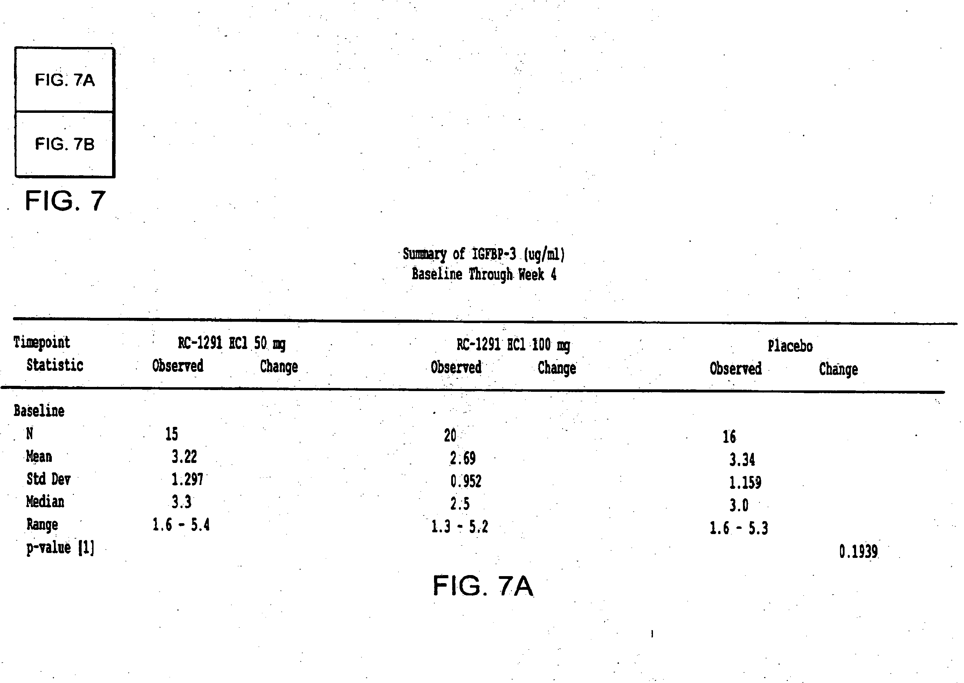

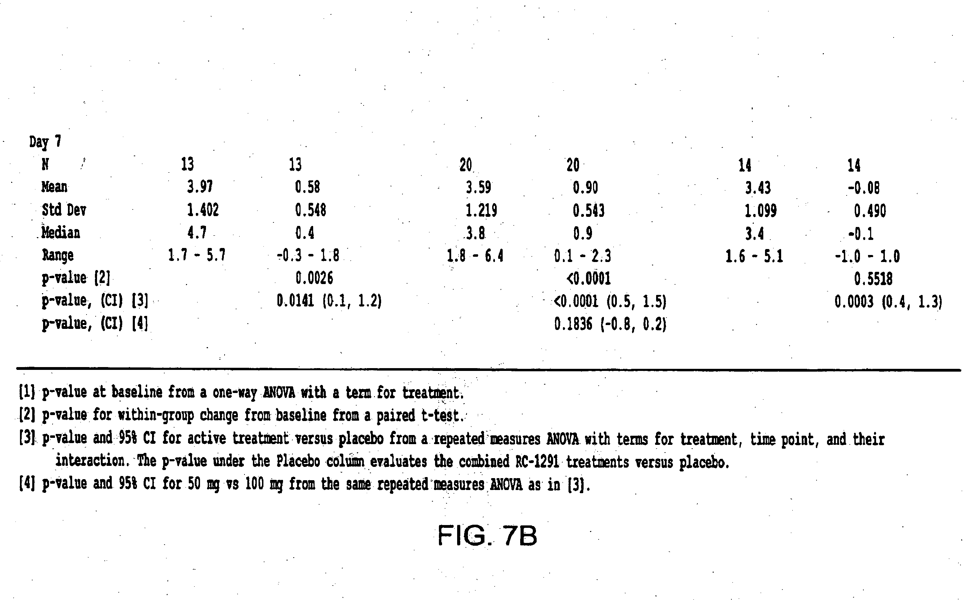

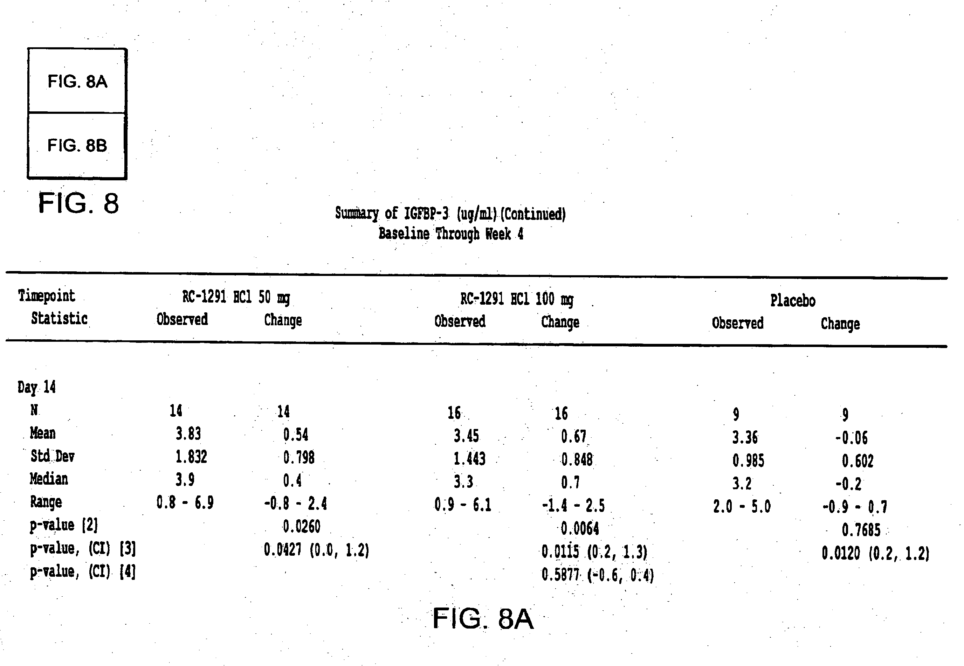

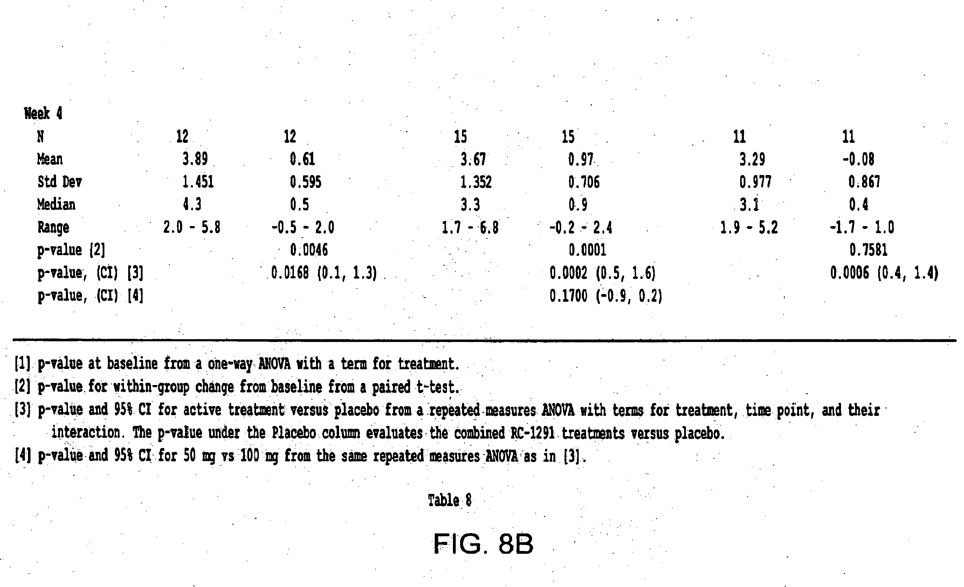

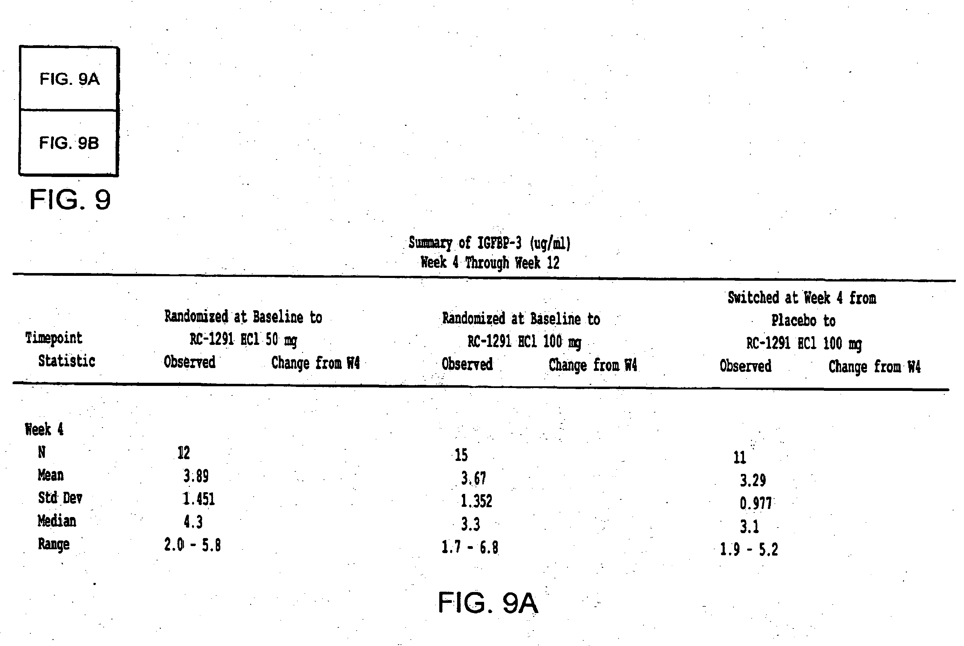

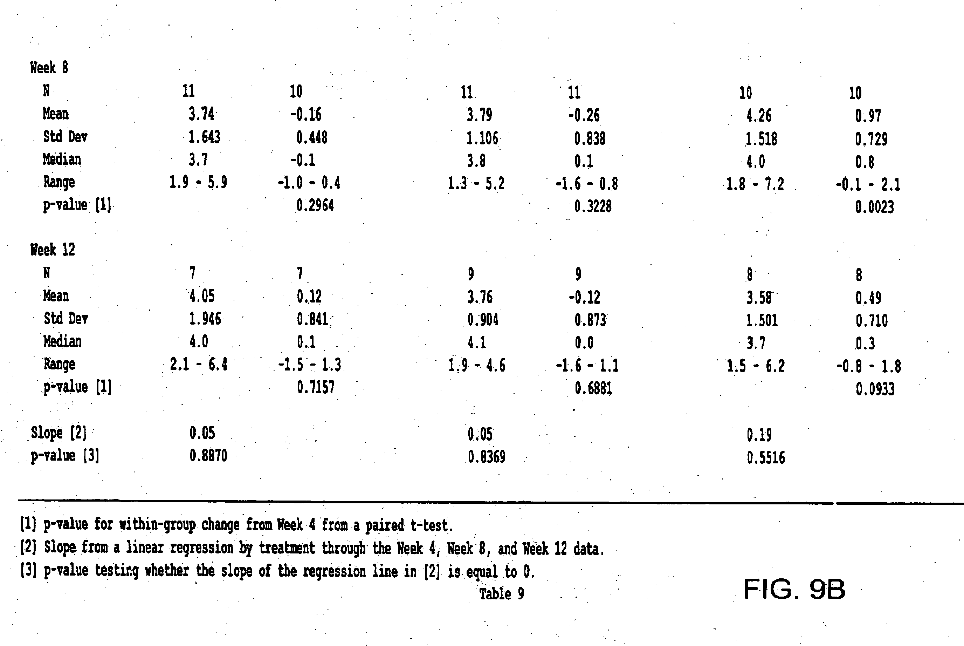

The summary of results is set forth in Table 3. Detailed results are presented in Tables 4-9 set forth in Figures 4-9 respectively. The results are presented graphically in Figures 2 and 3.

**Table 3**

| IGF-1 | | | | | | |
|---|---|---|---|---|---|---|
| | ng/mL | | | nM | | |
| Week | PBO | RC-1291 50 mg | RC-1291 100 mg | PBO | RC-1291 50 mg | RC-1291 100 mg |
| 0 | 0 | 0 | 0 | 0.00 | 0.00 | 0.00 |
| 1 | -5.86 | 40.69 | 80.25 | -0.77 | 5.32 | 10.49 |
| 2 | 22.33 | 57 | 76.25 | -2.92 | 7.45 | 9.97 |
| 4 | 16.82 | 46.5 | 73.4 | -2.20 | 6.08 | 9.60 |
| | | | | | | |
| | | | | | | |

| IGFBP-3 | | | | | | |
|---|---|---|---|---|---|---|
| | ng/mL | | | nM | | |
| Week | PBO | RC-1291 50 mg | RC-1291 100 mg | PBO | RC-1291 50 mg | RC-1291 100 mg |
| 0 | 0 | 0 | 0 | 0.00 | 0.00 | 0.00 |
| 1 | -80 | 580 | 900 | -2.78 | 20.19 | 31.32 |
| 2 | -60 | 540 | 670 | -2.09 | 18.79 | 23.32 |
| 4 | -80 | 610 | 970 | -2.78 | 21.23 | 33.76 |

The results presented in this Example demonstrate that RC-1291 raised the levels of both IGFBP-3 and IGF-1 in a statistically significant manner as compared to a placebo. The effects of RC-1291 on IGFBP-3 were 2 to 3 fold greater, on a molar basis, than on IGF-1.

Accordingly, the results presented in these Examples demonstrate that growth hormone secretagogues such as RC-1291 effectively raise the level of IGFBP- 3 relative to IGF-1 when administered to a subject and therefore will be useful for the treatment of cell proliferative disorders, e.g., cancer.

### Example 3: Treatment of Cancer Cachexia Using Growth Hormone Secretagogues

The placebo controlled, randomized studies described above were used to investigate the ability of growth hormone secretagogues to treat subjects having cancer cachexia.

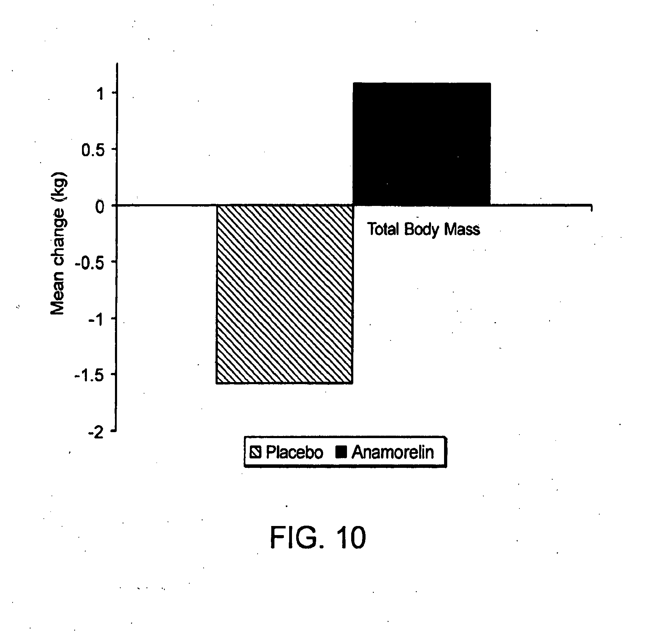

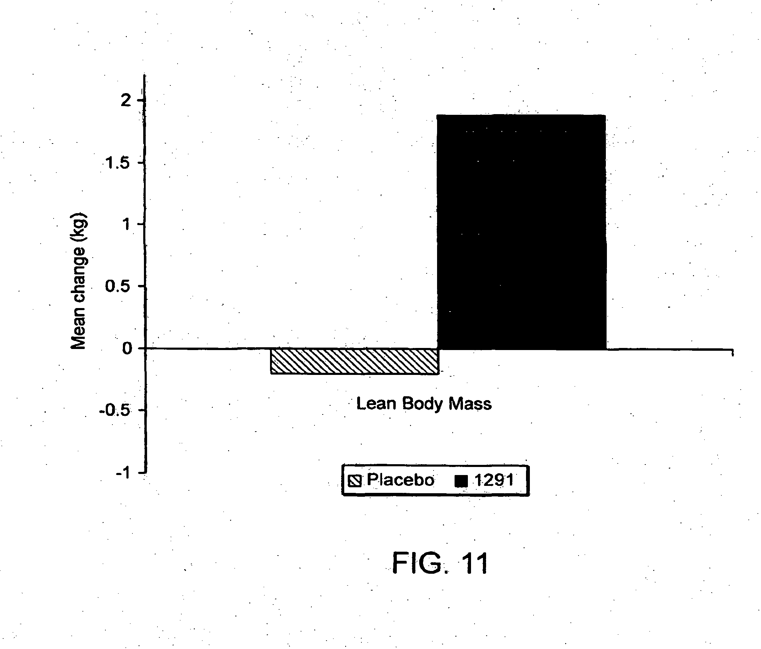

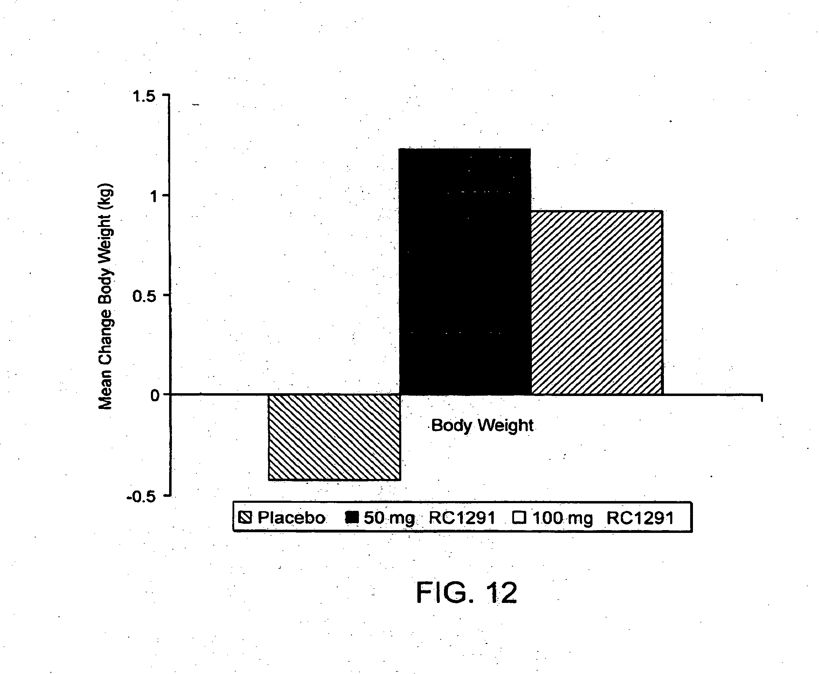

The results are presented in Figures 10, 11, and 12. Total body mass and lean body mass were measured for subjects described in Example 1 (see figures 10, and 11, respectively.) Lean body mass and total body mass were measured by dual- energy x-ray absorptiometry (DEXA). Total body weight was measured for subjects described in Example 2 (see, Figure 12).

The results presented in Figures 10, 11 and 12 demonstrate that subjects administered RC-1291 (Anamorelin) not only halted the change in lean body mass and total body weight, but that these subjects gained lean body mass and total body weight.

## Claims

1. A growth hormone secretagogue for use for treating cancer in a subject, wherein the growth hormone secretagogue is represented by the structural formula III 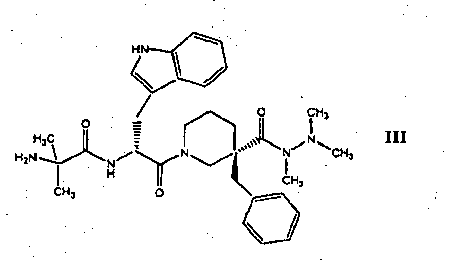 or a pharmaceutically acceptable salt, solvate or hydrate thereof.

2. The growth hormone secretagogue for use as in claim 1, wherein the cancer is a solid-tumor cancer.

3. The growth hormone secretagogue represented by the structural formula III, as defined in claim 1, for use for treating lung, colon or rectal cancer or for treating a subject having the risk of developing lung, colon or rectal cancer.

4. A composition for the use according to claims 1-3 comprising the growth hormone secretagogue of formula III and further comprising one or more additional anti-cancer agents.

5. The growth hormone sercretagogue for the use according to claims 1-3, wherein the growth hormone secretagogue is provided in dosages of between 25 mg/day - 150 mg/day; optionally wherein the growth hormone secretagogue is provided in dosages of between 50 mg/day -100 mg/day; particularly wherein the growth hormone secretagogue is provided in dosages of 50 mg/day.

6. A pharmaceutical composition for use for treating cancer in a subject, comprising a growth hormone secretagogue, one or more additional anti-cancer agents and a pharmaceutically acceptable carrier, wherein the growth hormone secretagogue is represented by the structural formula III as defined in claim 1.

7. The pharmaceutical composition for use as in claim 6, comprising between 10 mg to 150 mg of the growth hormone secretagogue, optionally 25 mg to 125 mg of the growth hormone secretagogue, particularly 25 mg to 100 mg of the growth hormone secretagogue, more particularly 10 mg to 50 mg of the growth hormone secretagogue, and specifically 50 mg to 100 mg of the growth hormone secretagogue.

## Patentansprüche

1. Wachstumshormon Sekretagogum zur Verwendung bei der Behandlung von Krebs bei einem Probanden, wobei das Wachstumshormon Sekretagogum durch die Strukturformel III dargestellt ist 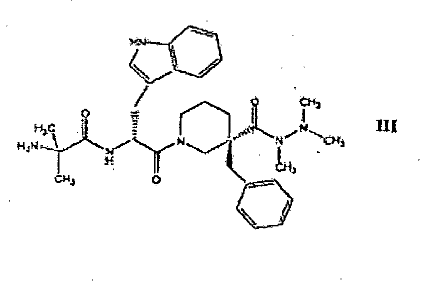 oder ein pharmazeutisches annehmbares Salz, Solvat oder Hydrat davon.

2. Wachstumshormon Sekretagogum zur Verwendung nach Anspruch 1, wobei der Krebs ein Krebs in Form eines soliden Tumors ist.

3. Wachstumshormon Sekretagogum, dargestellt durch die Strukturformel III, wie in Anspruch 1 definiert, zur Verwendung bei der Behandlung eines Lungen-, Kolon- oder Rektumkrebses oder zur Behandlung eines Probanden mit dem Risiko, einen Lungen-, Kolon- oder Rektumkrebs zu entwickeln.

4. Zusammensetzung zur Verwendung nach den Ansprüchen 1-3, umfassend das Wachstumshormon Sekretagogum der Formel III und weiter umfassend ein oder mehrere zusätzliche Antikrebsmittel.

5. Wachstumshormon Sekretagogum zur Verwendung nach den Ansprüchen 1-3, wobei das Wachstumshormon Sekretagogum in Dosierungen von 25 mg/Tag - 150 mg/Tag vorgesehen ist; wobei wahlweise das Wachstumshormon Sekretagogum in Dosierungen von 50 mg/Tag - 100 mg/Tag vorgesehen ist; wobei insbesondere das Wachstumshormon Sekretagogum in Dosierungen von 50 mg/Tag vorgesehen ist.

6. Pharmazeutische Zusammensetzung zur Verwendung bei der Behandlung von Krebs bei einem Probanden, umfassend das Wachstumshormon Sekretagogum, eines oder mehrere zusätzliche Antikrebsmittel und einen pharmazeutisch annehmbaren Träger, wobei das Wachstumshormon Sekretagogum durch die Strukturformel III nach Anspruch 1 dargestellt ist.

7. Pharmazeutische Zusammensetzung zur Verwendung nach Anspruch 6, umfassend 10 mg bis 150 mg des Wachstumshormons Sekretagogum, wahlweise 25 mg bis 125 mg des Wachstumshormons Sekretagogum, insbesondere 25 mg bis 100 mg des Wachstumshormons Sekretagogum, besonders bevorzugt 10 mg bis 50 mg des Wachstumshormons Sekretagogum, und speziell 50 mg bis 100 mg des Wachstumshormons Sekretagogum.

## Revendications

1. Sécrétagogue d'hormone de croissance pour une utilisation dans le traitement du cancer chez un sujet, dans lequel le sécrétagogue d'hormone de croissance est représenté par la formule développée III 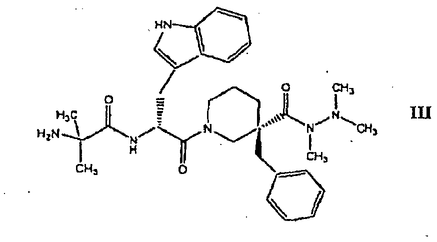 ou un sel, solvate ou hydrate pharmaceutiquement acceptable de celui-ci.

2. Sécrétagogue d'hormone de croissance pour une utilisation comme dans la revendication 1, dans lequel le cancer est un cancer de type tumeur solide.

3. Sécrétagogue d'hormone de croissance représenté par la formule développée III, telle que définie dans la revendication 1, pour une utilisation dans le traitement du cancer du poumon, du côlon ou rectal ou pour le traitement d'un sujet présentant le risque de développer un cancer du poumon, du côlon ou rectal.

4. Composition pour l'utilisation selon les revendications 1 à 3 comprenant le sécrétagogue d'hormone de croissance de formule III et comprenant en outre un ou plusieurs agents anti-cancer additionnels.

5. Sécrétagogue d'hormone de croissance pour l'utilisation selon les revendications 1 à 3, dans lequel le sécrétagogue d'hormone de croissance se présente en dosages compris entre 25 mg/jour et 150 mg/jour; optionnellement dans lequel le sécrétagogue d'hormone de croissance se présente en dosages compris entre 50 mg/jour et 100 mg/jour ; en particulier dans lequel le sécrétagogue d'hormone de croissance se présente en dosages de 50 mg/jour.

6. Composition pharmaceutique pour une utilisation dans le traitement du cancer chez un sujet, comprenant un sécrétagogue d'hormone de croissance, un ou plusieurs agents anti-cancer additionnels et un vecteur pharmaceutiquement acceptable, dans lequel le sécrétagogue d'hormone de croissance est représenté par la formule développée III telle que définie dans la revendication 1.

7. Composition pharmaceutique pour une utilisation comme dans la revendication 6, comprenant de 10 mg à 150 mg du sécrétagogue d'hormone de croissance, optionnellement de 25 mg à 125 mg du sécrétagogue d'hormone de croissance, en particulier de 25 mg à 100 mg du sécrétagogue d'hormone de croissance, plus particulièrement de 10 mg à 50 mg du sécrétagogue d'hormone de croissance, et spécifiquement de 50 mg à 100 mg du sécrétagogue d'hormone de croissance.
